**Europäisches Patentamt**

**European Patent Office**

**Office européen des brevets**

(19)

(11) Publication number : **0 511 750 A1**

(12) # EUROPEAN PATENT APPLICATION

(21) Application number : **92303159.5**

(22) Date of filing : **09.04.92**

(51) Int. Cl.⁵ : **C12Q 1/68, C12P 19/34, C07H 21/04**

(30) Priority : **09.04.91 US 683441**
**26.03.92 US 858124**

(43) Date of publication of application :
**04.11.92 Bulletin 92/45**

(84) Designated Contracting States :
**AT BE CH DE DK ES FR GB GR IT LI LU MC NL PT SE**

(71) Applicant : **THE REGENTS OF THE UNIVERSITY OF CALIFORNIA**
**300 Lakeside Drive, 22nd Floor, Office of the President**
**Oakland, California 94612-3550 (US)**

(72) Inventor : **Weier, Heinz-Ulrich Gunter**
**1151 Vallerand Road**
**Tracy, California 95376 (US)**
Inventor : **Gray, Joe William**
**1921 11th Avenue**
**San Francisco, California 94116 (US)**

(74) Representative : **Harrison, David Christopher et al**
**MEWBURN ELLIS 2 Cursitor Street**
**London EC4A 1BQ (GB)**

(54) **Repeat sequence chromosome specific nucleic acid probes.**

(57)   A primer directed DNA amplification method to isolate efficiently chromosome-specific repeated DNA wherein degenerate oligonucleotide primers are used is disclosed. The probes produced are a heterogeneous mixture that can be used with blocking DNA as a chromosome-specific staining reagent, and/or the elements of the mixture can be screened for high specificity, size and/or high degree of repetition among other parameters. The degenerate primers are sets of primers that vary in sequence but are substantially complementary to highly repeated nucleic acid sequences, preferably clustered within the template DNA, for example, alpha satellite repeat sequences. The template DNA is preferably chromosome-specific. The probes of this invention can be used to determine the number of chromosomes of a specific type in metaphase spreads, in germ line and/or somatic cell interphase nuclei, micronuclei and/or in tissue sections. Also provided is a method to select arbitrarily repeat sequence probes that can be screened for chromosome-specificity, and probes produced in a PCR method using nondegenerate primers.

EP 0 511 750 A1

The United States Government has rights in this invention pursuant to Contract No. W-7405-ENG-48 between the United States Department of Energy and the University of California for the operation of Lawrence Livermore National Laboratory.

The cytogenetic analysis of human cells and tissue material is typically based on microscopic inspection of banded metaphase chromosomes [Buckton and Evans 1973]. Cell samples taken from human tumors, however, usually contain too few cells in metaphase, so that mitogens have to be used to stimulate cellular proliferation. In solid tumors, stimulation of cellular growth of interphase cells is especially difficult or cannot be achieved at all [Gahrton et al. 1980; Trent 1985; Knuutila et al. 1986]. Cytogenetic analyses by means of in situ hybridization with chromosome-specific nucleic acid probes facilitate the differentiation between tumor cells and normal cells by allowing the analysis of interphase nuclei. Such analyses reduce the time and labor required for preparation of metaphase chromosomes and minimizes selection that may occur during cell culture [Cremer et al. 1986; Pinkel et al. 1986; Hopman et al. 1988; Trask et al. 1988; Nederlof et al. 1989].

Cloned probes suitable for chromosome-specific hybridization have now been reported for more than two-thirds of the human chromosomes. Some of such probes are specific for human satellite III DNA sequences [Cooke and Hindley 1979; Berdize 1987; Weier et al. 1990]. However, most of such probes bind to alpha satellite DNA found at or near the chromosome centromeres (pericentromeric) [Manuelidis 1978; Willard and Waye 1987]. The alphoid DNA sequences are comprised of tandemly repeated monomers of about 171 base pairs (bp) [Wu and Manuelidis 1980]. Certain parts of the 171 bp alphoid monomers appear to be conserved among all human chromosomes. Others, possibly organized as higher order repeats, show substantial chromosome-specific variation and may be used as the target of chromosomespecific probes [Devilee et al. 1986; Jorgensen et al., 1986; Murray and Martin 1987; Willard and Waye 1987]. Some authors have suggested that the chromosome-specificity is associated with organization of individual monomers in higher order repeats [waye et al., 1987a and 1987b; Willard and Waye 1987; Hulsebos et al. 1988]. However, there is evidence that some monomers are sufficiently specific and so highly repeated that they can be used as a hybridization target for interphase chromosome enumeration [Meyne and Moyzis 1989].

Many of the probes reported so far allow ready analysis of chromosome copy number [Choo et al. 1990]. Others, however, show considerable cross-hybridization with non-target chromosomes and may require hybridization at elevated levels of stringency [Waye et al. 1987b; Devilee et al. 1988]. Under such conditions, signal intensities often decrease, so that such probes cannot be used in critical applications, for-example, when hybridizing to highly condensed sperm chromatin [Wyrobek et al. 1990] or tissue sections wherein probe diffusion is poor [Emmerich et al. 1989] or when the hybridization target cannot be tightly controlled due partially to degradation of the cellular material.

Important probe parameters besides high specificity for the target chromosome type are the size of the probe molecule and the extent of the hybridization target area measured in base pairs (bp). For example, individual probe molecules may need to be of a size that favors diffusion into densely packed chromatin of sperm. Relatively short probe molecules that are complementary to highly reiterated DNA target sequences, such as the repeated satellite DNA, enable high signal intensities through binding of a large number of probe molecules to the target DNA without jeopardizing specificity. The preferred probes render highest possible signal-to-noise ratio.

The instant invention provides a primer directed DNA amplification method using the polymerase chain reaction (PCR) [Saiki et al. 1988b] with degenerate primers as an efficient means to isolate chromosome-specific repeated DNA. In the absence of any a priori knowledge other than the type of DNA repeat, for example, alpha satellite DNA, the methods of this invention allow the generation of chromosomespecific repeat sequence probe DNA. Disclosed are representative probes for human chromosome-specific alphoid DNA that have high specificity with high signal intensities in in situ hybridization experiments.

Weier et al. (1990) described the use of in vitro DNA amplification for production of double-stranded, biotinlabeled DNA probes. In that article a 124 bp segment of the Y chromosome-specific 3.4 kb repeat was amplified from human genomic DNA using PCR with nondegenerate primers.

Koch et al. (1989) disclosed a DNA analysis method called Primed Amplification Labeling (PAL) in which biotinlabeled hybridization probes are produced in a polymerase chain reaction (PCR), in which two synthetic oligonucleotide primers anneal within the same alphoid monomer. The instant method differs from the Koch et al. PAL method in several substantial ways. The primers used by Koch et al. are different from those of the instant invention, not only in the location of the primer annealing sites within the consensus monomer and the direction of primer extension, but, more importantly, the Koch et al. primers are nondegenerate. Thus, the amplification scheme described by Koch et al. is likely to amplify a rather limited number of different alphoid DNA sequences, and under typical PCR conditions, would not allow amplification of the cloned DNA fragments, for example, those in pBS609-51 and pBS609-52 discussed infra which have base pair mismatches.

Probe DNA molecules prepared according to the methods of this invention were cloned and analyzed by a combination of in vitro DNA amplification, dideoxynucleotide sequencing and in situ hybridization. Probes were screened for specificity, repeat content and size, among other parameters. The methods of this invention produce probes and collections of probes to highly repeated sequences such that the signal is much brighter and stronger than from a cloned repeat sequence probe that was prepared by conventional techniques.

Described herein are primer directed DNA amplification methods using degenerate primers to isolate efficiently chromosome-specific repeated DNA. The methods of this invention provide for the generation of chromosome-specific repeat sequence nucleic acid probes. Representative probes of this invention are repeat sequence probes for human chromosome-specific alphoid DNA.

Methods are provided to prepare chromosome-specific repeat sequence nucleic acid probes comprising:

binding a first set of degenerate oligonucleotide primers to repeat sequence units in template DNA that is chromosome-specific;

binding a second set of degenerate oligonucleotide primers to said repeat sequence units such that each of the 5′ ends of said first set of primers faces a 5′ end of one of said second set of primers, and the binding sites of said first set of primers are within a distance of from about 20 bp to about 5 kilobases (kb) of the binding sites of said second set of primers; and

amplifying the template DNA by a polymerase chain reaction (PCR) method between and including the first and second set of primers to produce chromosome-specific repeat sequence nucleic acid probes. Those probes can be prepared to be specific for any chromosomes that have repeat sequences. Preferably, those probes are specific for human chromosomes 1 through 22, X and Y.

Preferably, the repeat sequence units are clustered and more preferably, said clustered repeat sequence units are alphoid monomers. Representative preferred degenerate primers are WA1 and WA2 and/or WA11 and WA12 as shown in Table I, among others. Preferably, the template DNA is chromosome-specific DNA; preferably, the chromosome-specific template DNA has been flow-sorted, isolated by microdissection or by density gradients or is in a hybrid cell.

The probes can be labeled by performing the amplification step, at least in the later PCR cycles, in the presence of modified dNTPs. Alternatively, the probes can be labeled after completion of the PCR reaction by chemical or enzymatic modification of the PCR products.

Further disclosed are primers for preparing the probes of this invention, and the probes themselves. Still further disclosed are methods of using the probes of this invention. In these methods, unlabeled blocking DNA, for example, human genomic DNA, Cot 1 DNA and/or human alphoid DNA, can be used to reduce crosshybridization.

The chromosome-specific repeat sequence probes once separated from the genomic DNA, as a heterogeneous mixture of amplification products, can be screened for high specificity and other desirable probe parameters. They can be further amplified by a variety of methods including PCR and related methods. Cloned probes can be screened, for example, by gel electrophoresis for appropriate sizes. Selected clones are preferred templates for generating highly specific repeat sequence probes.

The probes of this invention are useful in enumerating specific chromosomes in interphase nuclei (both of germ line cells and somatic cells), micronuclei, metaphase spreads and/or tissue sections. Representative probes of this invention can be used to stain the pericentromeric and/or centromeric regions of specific chromosomes, for example, any of the human chromosomes.

Further, a method to select repeat probes arbitrarily is herein disclosed wherein a degenerate primer Jun1 which binds to DNA sequences having the repeat unit CAGG, is used in a PCR reaction with genomic and/or chromosome-specific DNA as template.

Further disclosed are probes prepared by nondegenerate primers as follows: a human centromere-specific nucleic acid probe for human chromosomes 21 and 13 prepared from chromosome 21-specific template DNA by a polymerase chain reaction (PCR) method wherein the primers are W21R1 and W21R2; a human chromosome X-specific repeat sequence probes prepared from human chromosome X-specific template DNA by a PCR method wherein the primers are WXR1 and WXR2; a human chromosome 9-specific repeat sequence probe prepared from human chromosome 9-specific template DNA by a PCR method wherein the primers are WYR9 and WYR10; and a mouse centromere-specific repeat sequence probe prepared from mouse total genomic template DNA by a PCR method wherein the primers are WGS1 and WGS2.

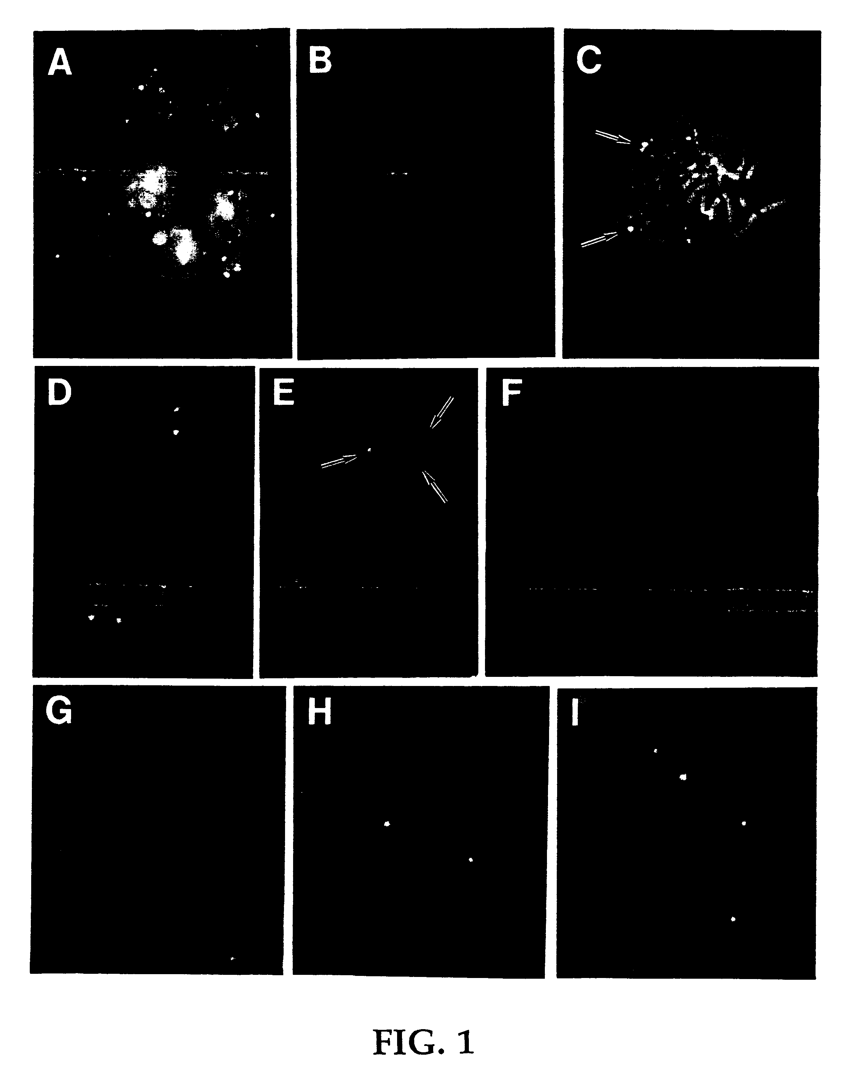

Figure 1 shows the in situ hybridization of chromosome 10-specific probe DNAs. The biotinylated probe molecules in (A), (C), and (F)-(H) were visualized with avidin-FITC (green fluorescence) and the AAF-labeled DNA probe shown in (D) and (E) were detected with FITC-conjugated antibodies (green fluorescence). DNA was counterstained with PI (red fluorescence). The digoxigenin labeled DNA probes in (B) and (I) were visualized with FITC-conjugated antibodies (green fluorescence). The biotinylated probe in (B) was detected using avidin-Texas Red (red fluorescence). The domains of bound probe appear yellow due to the superposition of red and green fluorescence in (A) and (C)-(I).

3

A. A biotinylated probe DNA was generated by PCR using isolated human chromosomes as amplification template. Metaphase spreads from lymphocytes from a normal donor showed hybridization of probe DNA with repeated DNA probe DNA in the centromeric region of chromosomes 10. There is, however, binding of probe molecules to repeated DNA at or near the centromeres of other chromosomes.

B. Interphase cell nuclei were hybridized with a mixture containing the biotinylated, chromosome 10 alpha satellite DNA probe shown in (A) in combination with a digoxigenin labeled probe for DNA of the Alu repeat family. The nuclei are shown with binding of the biotinylated alpha satellite DNA to numerous sites (red fluorescence).

C. The probe specificity was increased by blocking the binding of less specific DNA fragments. Metaphase spreads from lymphocytes from a normal donor showed that the biotinylated probe DNA hybridizes preferentially with repeated DNA in the centromeric region of chromosomes 10 (arrows) when the hybridization mixture contained unlabeled total human alphoid DNA.

D. Hybridization of the AAF-labeled DNA to a single cell suspension of human kidney cells. The cell material was obtained by enzymatic digestion of tissue adjacent to an aneuploid tumor. Interphase cell nuclei with the normal complement of 2 copies of chromosomes 10 showed two bright spots delineating domains of chromosome 10-specific alphoid DNA, when the degenerate probe DNA was hybridized in the presence of human genomic DNA as blocking agent.

E. Some of the cells in the preparation shown in (D) showed three domains of bound probe indicating the presence of an extra copy of chromosome 10 in those cells.

F. Biotinylated degenerate probe DNA was hybridized to deparaffinized serial sections of ovarian cancer tissue.

G. The clonal probe DNA pBS609-51 was biotinylated and hybridized without blocking DNA to serial sections from an ovarian tumor.

H. Metaphase spreads revealed exclusive binding of DNA from another clone (pBS609-13) to the centromeric region of human chromosome 10.

I. The digoxigenin labeled probe prepared from the bacterial clone pBS609-51 bound very specifically to DNA in the centromeric region of chromosomes 10 without signs of crosshybridization to alphoid DNA domains on other chromosomes. The red bands that can be observed along the chromosomes in this photograph were generated by simultaneous hybridization of a biotinylated Alu-repeat DNA probe, that was detected with avidin-Texas Red.

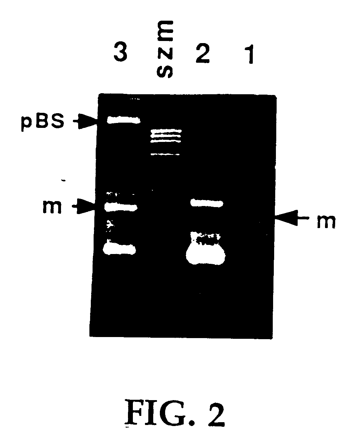

Figure 2 shows the size distribution of in vitro DNA amplification products using primers WA1 and WA2, and isolated human chromosome 10 as the amplification template; (lane 1) shows a single band in the monomer size interval ('m'). The size distribution of PCR products obtained with primers WA11 and WA12 shows additional small fragments (lane 2). The different DNA fragments can be found in Bam H1 digested plasmid DNA (lane 3). ('m' indicates inserts in the monomer size interval; 'pBS' indicates the linearized plasmid DNA.) The sizemarker DNA lane (szm) contains 400 ng of φX174 RF DNA/Hae III digest.

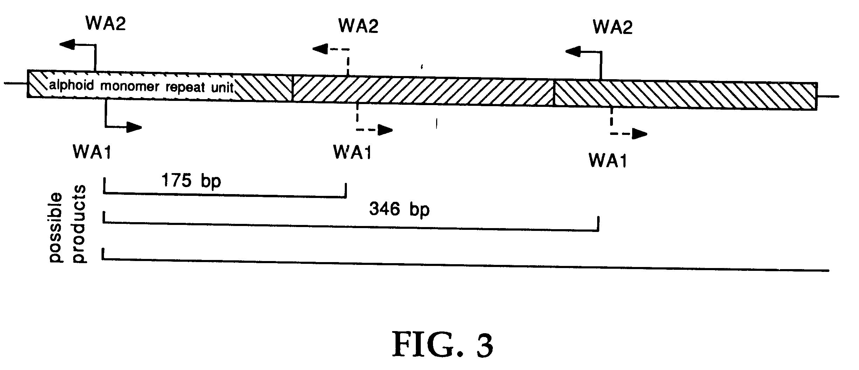

Figure 3 shows schematically the putative structure of alpha satellite DNA repeats and possible annealing sites of oligonucleotide primers. Arrows indicate the direction of primer extension by DNA polymerase. Shaded areas indicate stretches of alphoid DNA that have higher homology with the alphoid consensus sequence. The expected sizes of primer extension products using primers WA1 and WA2 in a PCR protocol are indicated at the bottom of the figure.

The following abbreviations are used herein:

## Abbreviations

| | | |
|---|---|---|
| A | — | adenine |
| AB | — | antibody |
| AAF | — | N-acetoxy-2-acetylaminofluorene |
| AMCA | — | 7-amino-4-methylcoumarin-3-acetic acid |
| bcip/npt- | | 5-bromo-4-chloro-3-indolylphosphate/nitroblue tetrazolium |
| Buffer A- | | Amplification buffer consisting of 10 mM Tris-HCl, pH 8.4 at 20°C; 1.5 mM $MgCl_2$; 50 mM KCl; 0.2 mM each of dATP dCTP, dGTP and dTTP [all dNTPs from Sigma Chem. Co. (St. Louis, MO (USA)] |
| bp | — | base pair |
| BRL | — | Bethesda Research Laboratories [Gaithersburg, MD (USA)] |
| C | — | cytosine |
| °C | — | degrees centigrade |
| DAPI | — | 4,6-diamidino-2-phenylindole |

| | | |
|---|---|---|
| dATP | – | 2'-deoxyadenosine 5'-triphosphate |
| dCTP | – | 2'-deoxycytidine 5'-triphosphate |
| dGPT | – | 2'-deoxyguanosine 5'-triphosphate |
| dITP | – | 2'-deoxyinosine 5'-triphosphate |
| DNA | – | deoxyribonucleic acid |
| dNTP | – | deoxynucleotide triphosphate |
| dTTP | – | 2'-deoxythymidine 5'-triphosphate |
| dUMP | – | 2'-deoxyuridine 5'-monophosphate |
| dUTP | – | 2'-deoxyuridine 5'-triphosphate |
| EB | – | ethidium bromide |
| EDTA | – | ethylenediaminetetraacetate |
| FA | – | formamide |
| FCM | – | flow cytometry |
| FITC | – | fluorescein isothiocyanate |
| G | – | guanine |
| g | – | gram, gravity |
| HPLC | – | high performance liquid chromatography |
| h | – | hour |
| IPTG | – | isopropylthio-beta-D-galactosidase |
| kb | – | kilobase |
| KCl | – | potassium chloride |
| LB | – | Luria-Bertani |
| M | – | molar |
| Mb | – | megabase |
| Mg | – | milligram |
| min | – | minute |

ml     —     milliliter

mm     —     millimeter

mM     —     milliMole

N     —     normal concentration

ng     —     nanogram

NP-40     —     non-ionic detergent commercially available from Sigma as Nonidet P-40 (St. Louis, MO)

nt     —     nucleotide

pBR     —     plasmid cloning vector available from BRL (Gibco/BRL Catalog and Reference Guide)

PBS     —     phosphate-buffered saline

pBS     —     Bluescribe plasmid cloning vector

pH     —     hydrogen ion concentration

PCR     —     polymerase chain reaction

PI     —     propidium iodide

PN buffer     —     mixture of 0.1 M $NaH_2PO_4$ and 0.1 M $Na_2HPO_4$, pH 8; 0.1% NP-40

PNM buffer     —     Pn buffer plus 5% nonfat dry milk (centrifuged); 0.02% Na azide

RCC     —     renal cell carcinoma

RT     —     room temperature

sec     —     seconds

SSC     —     0.15 M NaCl/0.015 M Na citrate, pH 7

T     —     thymine

Taq     —     Thermus aquaticus

TE     —     10 mM Tris-HCl, PH 7.5, 0.5 mM Na-EDTA

TR     —     Texas Red

Tris     —     tris (hydroxymethyl) aminomethane

EP 0 511 750 A1

```
ug        -     microgram

ul        -     microliter

um        -     micrometer

w/v       -     weight to volume

w/w       -     weight to weight

X-Gal     -     5-bromo-4-chloro-3-indolyl-beta-D-
                galactosidase
```

The following references are cited herein:
Baldini et al., Am. J. Hum. Genet., 46: 784-788 (1990);
Baldini and Ward, Genomics, 9: 770-774 (1991);
Berdize, Satellite DNA [Springer-Verlag, Berlin, Heidelberg, New York, Tokyo (1987)];
Brigati et al., Virology, 126: 32-50 (1983);
Buckton and Evans, Methods for the Analysis of Human Chromosome Aberrations [World Health Organization, Geneva (1973)];
Chamberlain, et al., Nucl. Acid Res., 16: 11141-11156 (1988);
Choo et al., Genomics, 7(2): 143-151 (June 1990);
Cooke and Hindley, Nucl. Acids. Res. 6: 3177-3197 (1979);
Cremer et al., Hum. Genet., 74: 346-352 (1986);
Devilee et al., Nucl. Acids Res., 14: 2059-2073 (1986);
Devilee et al., Genomics, 3: 1-7 (1988);
Emmerich et al., Lab. Invest., 61: 235-242 (1989);
Frommer et al., Chromosoma, 97: 11-18 (1988);
Gahrton et al., Blood, 56: 640-647 (1980);
Green and Olson, PNAS, 87: 1213-1217 (1990);
Guatelli et al., PNAS, 87: 1874-1878 (March 1990);
Gussow and Clackson, Nucl. Acids Res., 17: 4000 (1989);
Harper et al., Blut, 48: 33-43 (1981a);
Harper et al., PNAS, 78: 4458 (1981b);
Hopman et al., Histochem, 89: 307-316 (1988);
Hopman et al., Am. J. Path., 135: 1105-1117 (1989);
Hulsebos et al., Cytogenet. Cell Genet., 47: 144-148 (1988);
Johnson, Genomics, 6: 243 (1990);
Johnson and de C. Nogueira Araujo, J. Immunol. Meth., 43: 349-351 (1981);
Jorgensen et al., J. Mol. Biol. 187: 185-196 (1986);
Kievits et al., J. Virol. Methods, 35 (3): 237-286 (1991);
Knuutila et al., New Engl. J. Med., 314: 865-869 (1986);
Koch et al., Chromosoma, 98: 259-265 (1989);
Kunicka et al., Cancer Res., 47: 3942-3947 (1987);
Landegent et al., Exp. Cell Res., 153: 61-72 (1984);
Lizardi et al., BioTechnology, 6: 1197-1202 (1988);
Lo et al., Nucl. Acids Res., 16: 8719 (1988);
Lo et al., "Incorporation of biotinylated dUTP", In: PCR Protocols (Innis et al. eds.) [Academic Press, San Diego, pp. 113-118 (1990)];
Maniatis et al., Molecular cloning: A Laboratory Manual [Cold Spring Harbor: Cold Spring Harbor Laboratory (1986)];
Manuelidis, Chromosoma, 66: 23-32 (1978);
Meltzer et al., Nature-Genetics,                    :                    (1992)
Meyne and Moyzis, Genomics, 4: 472-478 (1989);
Moroi et al., PNAS, 77: 1627-1631 (1980);
Murano et al., Clin. Genet., 39: 68-74 (1991);
Murray and Martin, Gene, 57: 255-259 (1987);

8

Nakahori et al., Nucl. Acids Res., 14: 7569-7580 (1986);
Nakamura et al., Science, 235: 1616 (1987);
Nederlof et al., Cancer Genet. Cytogenet., 42: 87-98 (1989);
Nelson et al., PNAS, 86: 6686-6690 (1989);
Pinkel et al., "Cytogenetic analysis by in situ hybridization with fluorescently labeled nucleic acid probes. In: Cold Spring Harbor Symposia on quantitative Biology, Vol. LI [Cold Spring Harbor, Cold Spring Harbor Harbor Laboratory, pp. 151-157 (1986)];
Pinkel et al., PNAS, 85: 9138-9142 (1988);
Saiki et al., Science, 235: 1616 (1988a);
Saiki et al., Science, 239: 487-491 (1988b);
Sanger et al., PNAS, 74: 5463-5467 (1977);
Saunders et al., Nucl. Acids Res., 17: 9028 (1990);
Trask et al., Hum. Genet. 78: 251-259 (1988);
Trask et al., Somatic Cell Mol. Genet., 17: 117-136 (1991);
Trent, Breast Cancer Res. Treatm., 9: 221-229 (1985)
Van Dilla et al., Bio/Technology 4: 537-552 (1986);
Vissel and Choo, Genomics, 5: 407-414 (1989);
Walker et al., PNAS, 89: 392-396 (1992);
Waye and Willard, Nucl. Acids Res., 15: 7549-7569 (1987);
Waye et al., Nucl. Acids Res., 13: 2731-2743 (1985);
Waye et al., Molec. and Cellular Biol., 6: 3156-3165 (1986);
Waye et al., Chromosoma, 95: 182-188 (1987a);
Waye et al., Genomics, 1: 43-51 (1987b);
Weier and Gray, DNA 7: 441-447 (1988);
Weier and Rosette, Nucl. Acids Res., 16: 11836 (1988);
Weier and Rosette, BioTechniques, 8: 252-257 (1990);
Weier et al., J. Histochem. Cytochem., 38: 421-426 (1990)
Weier et al., Hum. Genet, 87: 489-490 (1991a);
Weier et al., Chromosoma, 100: 371-376 (1991b);
Weier et al., BioTechniques, 10: 498-505 (1991c);
Willard and Waye, Trends in Genet. 3: 192-198 (1987);
Wu and Manuelidis, J. Mol. Biol., 142: 363-386 (1980); and
Wyrobek et al., Mol. Reprod. Devel., 27: 200-208 (1990).

The present invention describes probes, and a methods for making them, which are specific for repeat sequences on chromosomes. Although most of the examples herein are directed to human chromosomes, the methods of this invention can be used to produce repeat sequence probes of high specificity for other species, preferably mammalian.

Repeat sequences can occur in the genome in multiple copies which range from two to hundreds of thousands of copies. The copies of a repeat sequence may be clustered in one or more locations such as near the centromere, telomere or variable number tandem repeat (VNTR) [Nakamura et al. 1987]. Copies of the repeat sequences may be clustered, or they may be interspersed, that is, distributed on one or more chromosomes or throughout a genome.

Various families of satellite DNA have been described, including those referred to as alpha, beta (also termed the Sau 3A family) and gamma satellites or another group, human satellites I, II, III and IV. A common feature of these satellite DNA structures is that they are composed of small repeated units, for example, the alpha satellite repeat unit in humans is approximately 171 bp in length. Representative clustered repeat sequences are alpha satellites; beta satellites; satellites I, II, III and IV; and the 39 bp repeat that maps to 1p36.

## Primer Sequences

Typically, probe primer sequences are chosen which not only flank the desired probe sequence, but are included within the probe sequence itself in order to avoid the requirement for separation. In one preferred embodiment of the invention, the binding sites of one set of degenerate primers are within a distance of from about 20 bp to about 5 kb of the binding sites of said second set of primers. Primers may range from 8 to 100 bp in length, more preferably 20-30 bp. When very small primers are selected, annealing is accomplished at very low temperatures, often as low as 4°C. The most useful primers are those which bind only to areas where the selected repeat sequences will be primed to avoid obtaining undesired sequences in the amplification products.

The appropriate primer for the desired repeat sequence probe is made by selecting and making a set of degenerate oligonucleotide primers which contain a variety of different nucleotide sequences, each of which is slightly less than homologous to a specific stretch of nucleic acid of a known repeat sequence on the target nucleic acid. They may differ by one or more bases from the binding sequence on the target DNA. For example, the degeneracy of the primer sequence, introduced by the chemical oligonucleotide synthesis, is eight to 16-fold, with a 12-fold degeneracy preferred, in particular. Additional restriction enzyme recognition sites may be added to the primer to facilitate further molecular cloning. The sequences of oligonucleotide primers used in the examples and other nondegenerate primers are shown in Table I.

## Table I   Synthetic oligonucleotides used in the examples

| | |
|---|---|
| WA1[2] | GAAGCTTA($^A$/$_T$)($^C$/$_G$)T($^C$/$_A$)ACAGAGTT($^G$/$_T$)AA |
| WA2[2] | GCTGCAGATC($^A$/$_C$)C($^A$/$_C$)AAG($^A$/$_T$/$_C$)AGTTTC |
| WA11 | CCC GGA TCC CTG CAG AAG CTT A(A/T)(C/G)T(C/A) ACA |
| WA12 | CCC GGA TCC AAG CTT CTG AGA TC (A/C)C(A/C) AA |
| WGS1 | CCC AAG CTT GAA ATG TCC ACT |
| WGS2 | CCC AAG CTT TTT CTT GCC ATA |
| WBS2 | CTC GGA ATT AAC CCT CAC TAA AGG |
| WBS4 | GAA TTG TAA TAC GAC TCA CTA TAG |
| WXR1 | TCG AAA CGG GTA TAT GCT CAC GTA AAA |
| WXR2 | AAG ACA GTT TCA AAA CTG CTC CAT CAA |
| W21R1 | GGA TAG CTT AAC GAT TTC GTT GGA AAC |
| W21R2 | CAA ACG TGC TCA AAG TAA GGG AAT G |
| WYR9 | ATG GAA TTG AAT GGA ACG GAA TAG AGT |
| WYR10 | CGA TTC CAT TCA ATT CGA GAC CAT TCT |

[1] All oligonucleotides are listed from 5' to 3'.

[2] Degenerate primer.

Probes for the repeat sequences of human centromeres can be made by selecting degenerate oligonucleotide primers which are homologous to two regions of the 171 bp alpha satellite (alphoid) repeat sequence. That repeat sequence is conserved in all human chromosomes. Specifically, in a preferred example, two primers, WA1 and WA2 were produced which bind respectively to the alpha satellite repeat consensus sequence at bp positions 37-52 and 10-26. The consensus sequence for alpha satellite DNA was published by Waye and Willard (1987).

The degenerate primers for the alpha satellite repeat sequences are placed so that the 5'-ends face each other when annealed to the genomic DNA template. The minimal product size expected is 175 bp, based on the distance between the annealing sites (Fig. 3). During amplification of longer products, multiple 170 bp repeat sequences, may occur between primer and annealing sites when the individual monomeric repeat units have a lower degree of homology with the consensus sequence, so that the primers can not anneal in each repeat monomer. In that example, the primer annealing sites are separated by one or more repeat units. As such, the DNA segments amplified contain DNA segments of 175 bp size plus one or more multiples of the 171 bp repeat unit.

The primer sequences listed herein in Table I may not be suitable far amplifying all naturally occurring alphoid sequences. However, the degeneracy of the primers introduces a maximum flexibility in using the amplification scheme, and ones of ordinary skill in the art would know how to prepare various primers according to this invention for different chromosomes, as for example, the Y chromosome.

As indicated by the sequence analyses of the clones pBS609-51 and -52 as discussed infra in Example 1 and shown in Table II, different primer sequences are involved in the probe amplification process, and the primer sequences differ from the consensus sequence. Different primer sequences from the pool of primer sequences represented by WA1 and WA2 may anneal to individual stretches of template DNA as schematically represented in Figure 3; thus, the complexity of the amplification products is increased.

## Arbitrarily Selected Repeat Probes

If annealing sites are part of an inverted repeat, the reaction may be performed with one primer. When the annealing sites have the same orientation along the chromosome (i.e. pter to qter direction), one or more pairs of oligonucleotide primers can be used. The primer molecules can be complementary to each other, as long as annealing to each other during one PCR cycle does not interfere with annealing of the primer molecules during subsequent cycles. Oligonucleotides that bind to sequences as small as 4-10 bp in length can be used as primers.

A preferred primer of this invention for arbitrarily priming is termed Jun1 which has the following 29 nucleotides:

5'-CCCAAGCTTGCATGCGAATTCXXXXCAGG-3'

wherein each X stands for any of the 4 DNA bases--A, C, G or T. Therefore, said primer has 256 possible combinations by changing the 4X positions (4 x 4 x 4 x 4 = 256). Those X positions are 5' to the 4 base recognition sequence that was found to be conserved at spliced junctions. At the 5' end of Jun1 is the triplet sequence CCC which serves as a C/G clamp to keep the hybridized strands together. The next 18 nucleotides provide a number of different restriction enzyme recognition sites.

The Jun1 primer can be used in combination with human genomic DNA or chromosome-specific DNA, as for example, flow-sorted chromosomes, to amplify chromosomespecific repeated DNA. When the Jun1 primer is used, the 39 bp repeat that maps to 1p36 is preferentially amplified.

## Synthesis of the Probe

The synthesis of the probe may be accomplished by conventional polymerase chain reaction (PCR) process. The mechanics of PCR are explained in Saiki et al., Science 230: 1350 (1985) and U.S. Patent Nos. 4,683,195 and 4,683,202 (both issued July 28, 1987) and 4,800,159 (issued January 24, 1989). A PCR adapter-linker method is explained in Saunders et al. (1990); Johnson (1990) and PCT 90/00434 (published August 9, 1990). Another PCR method employing a mixture of primers is described in Meltzer et al., "Generation of Region Specific Probes by Chromosome Microdissection: A Novel Approach to Identify Cryptic Chromosomal Rearrangements," Nature--Genetics,                               :                               (1992).

Once the methods of this invention employing PCR using degenerate primers has separated the repeat sequence probe sequences from the genomic DNA, many different methods can be used to prepare large quantities of the probes for screening, sequencing and/or use in in situ hybridization experiments. For example, the PCR amplification products can be further amplified by PCR processes and/or molecularly cloned in a variety of vectors as illustrated in Example 2.

In a cloning stratety, primers that anneal to flanking vector sequences are advantageous, because each primer can be extended into a probe molecule, and a single pair of vector primers can be used to amplify different kinds of inserts. Once the insert has been sequenced, one can synthesize nondegenerate primers that match the 5' ends of the insert. Then, the two pairs of primers can be used as nested sets for improved specificity (Lo et al. 1990; Weier et al. 1991c) in a step reaction similar to the probe synthesis scheme described above. Automated programmable PCR methods may be used for large scale probe synthesis such as that using thermostable DNA polymerase which was described by Weier and Gray (1988).

Newer techniques using the Q-beta replicase system [Lizardi et al. 1988] or ligase chain reaction, as commercialized by BioTechnica International Inc. [Cambridge MA (USA)], could be used to make copies of the desired probe or probes. Further, an isothermal in vitro amplification of those DNA sequences using a restriction enzyme DNA polymerase system could be used as described in Walker et al. (1992), Guatelli et al. (1990), and Kievits et al. (1991).

## Template DNA

Limiting the complexity of the template DNA is important for chromosome-specificity of the amplification products prepared according to this invention. Preferably, for the generation of probes of this invention, chromosomes isolated by flow sorting are used as the template DNA. The chromosome-specific DNA may also be isolated from a hybrid cell, isolated by microdissection or by density gradients, or by other means. Further, the chromosome-specific template DNA can be chromosome-specific libraries.

## Labeling of Probes

The probes of this invention can be labeled during the PCR amplification in the presence of appropriately modified dNTPs, or they can be labeled after completion of the PCR reaction by chemical or enzymatic modification of the PCR products. Any of the various labeling techniques, direct or indirect, may be used to label probes, including but not limited to fluorescent chemicals, radioactive materials, chemical haptens, or enzymatic modifiers. More than one label can be used. Preferred modified dNTPs include but are not limited to biotin-11-dUTP; digoxigenin-11-dUTP; biotin derivatives of dATP; fluoresceinated-dUTP; rhodamin labeled derivatives of dUTP; rhodamin labeled derivatives of dCTP; hydroxy coumarin-labeled derivatives of dUTP; and resorufin-11-2′-dUTP. Preferred labels include but are not limited to AAF, sulfur and mercury.

The staining intensity achieved using the probes may be amplified with a variety of systems, including but not limited to fluorochrome conjugated avidin and/or labeled antibodies. DNA can be counterstained with DNA-specific dyes, including but not limited to DAPI, that fluoresce in different wavelength intervals dependent on the selected scheme for visualization of bound probe molecules.

In Situ Hybridization

As indicated in Example 1 below, the probes of this invention can be used for in situ hybridization to metaphase spreads, interphase nuclei and/or tissue sections. The interphase nuclei can be from germ line cells and/or somatic cells. The chromosomal material of micronuclei can also be a target of appropriately sized probes of this invention. Modifications of standard hybridization protocols as detailed in Pinkel et al. 1988 are used.

In Example 1, it is shown that centromeric repeated DNA can be selectively amplified using a pair of degenerate alpha satellite consensus primers. PCR reproduces naturally occurring variations in the base sequences between primer annealing sites by using oligonucleotide primers that anneal in the most conserved parts of the alphoid monomer repeat. The resulting probe DNA is a heterogeneous mixture of DNA fragments in different size ranges with a variety of nucleotide sequences. The degeneracy of the PCR-generated probe DNA may be advantageous during in situ hybridization compared to the use of conventional clonal DNA probes, because it allows deposition of a very dense array of probes along the chromosomal target.

As indicated in Weier et al. (1991a), separation of the chromosome 8-specific DNA prepared as described therein essentially as the chromosome 10-specific DNA of Example 1, into three different size classes, labeling and hybridizing them in situ indicated that crosshybridization was mostly due to the monomeric fraction, while the longer DNA fragments showed higher specificity for the target chromosome 8. The size distribution of chromosome 10 derived amplification products is very similar to those observed with chromosome 8-specific template DNA [Weier et al. 1991a], and similarly, the biotinylated probe DNA shows significant crosshybridization with other chromosomes that can be blocked by the addition of unlabeled total human alphoid DNA. That blocking DNA could also be total human genomic DNA and/or Cot 1 DNA.

The preferred ratio (w/w) for human alphoid blocking DNA to labeled probe of this invention is from about 2:1 to about 5:1. The preferred ratio (w/w) of total human genomic DNA to labeled probe of this invention is from about 10:1 to about 30:1, preferably about 25:1. The blocking DNA is used to inhibit less specific elements of the probe from binding.

Molecular cloning

The PCR methods of this invention using degenerate primers as indicated above produce a heterogeneous mixture of probes, some of which are more or less chromosomespecific. As indicated immediately above, blocking DNA is used to inhibit the less specific elements of the mixture from binding. Thus, the addition of the blocking DNA creates a chromosome-specific staining reagent from the heterogenous mixture.

As exemplified in Example 1, highly specific repeat sequence probes can be prepared by screening the heterogeneous mixture for highly specific probe elements. In a representative screening method, the probe elements can be molecularly cloned, and clones selected first that contain inserts in an appropriate size range. The appropriately sized elements are then labeled and screened by in situ hybridization experiments for chromosome-specificity. Also clones can be selected for those containing inserts that have highly repeated DNA which have more target sites and deliver stronger hybridization signals. There can be many variations of the screening protocol, but that presented in Example 1 is a preferred screening method.

Once a clone has been found to contain a highly specific repeat sequence, for example, as were the chromosome 10-specific clones pBS609-51 and pBS609-52, such a clone is a preferred template for PCR based probe production. Another useful aspect of probe generation by PCR from clonal templates is the fact that primers can be designed to incorporate some parts of the vector sequence into the probe (Lo et al. 1988; Weier and Rosette 1988). The non-homologous vector tail on the 5′-end of the probe molecules does, in most cases, not interfere with probe annealing during the hybridization (Frommer et al. 1988; Weier et al. 1991c), but might virtually enlarge the hybridization target and enhance signal intensity by binding an increased number of reporter molecules. Some preferred vectors include Bluescribe plasmids and more generally pUC derived plasmids. Preferred primers include but are not limited to WBS2, WBS4 (Table I), M13 forward and reverse sequencing primers, and primers that bind to T3 and T7 RNA polymerase promoters.

The following examples are for purposes of illustration only and are not intended to limit the scope of the invention in any way.

EXAMPLES

Example 1

Probe for Human Chromosome 10-specific Alpha Satellite DNA

a. Oligonucleotide primers

Oligonucleotide primers--WA1, WA2, WBS2 and WBS4--homologous to parts of the alpha satellite consensus sequence [Waye and Willard 1987] or the cloning vector pBS were synthesized using phosphoramidite chemistry on a DNA synthesizer [Applied Biosystems, Foster City, CA (USA), model 380B]. Synthesis and further purification of the oligonucleotides by $C_{18}$ reverse phase chromatography and HPLC were performed according to the specifications of the manufacturer [Waters Chromatography, Milford, MA (USA)].

The sequences of the oligonucleotide primers including their degenerate positions are shown in Table I. Primers WA11 and WA12 were deprotected by overnight incubation in 2N $NH_3$. The primers were then lyophilized, resuspended in water and ethanol precipitated [Maniatis et al. 1986]. All primers were prepared as 30 uM stock solutions in water and stored at -20°C.

The degenerate alpha satellite primers WA1, WA2, WA11, and WA12 carry non-homologous bases to facilitate molecular cloning of PCR products. Primers WA1 and WA2 carry a 5′ Hind III and Pst 1 recognition sequence, respectively. WA11 and WA12 carry short polylinker sequences that represent from 5′ to 3′ restriction enzyme recognition sites for Bam H1, Pst 1 and Hind III or Bam H1, Hind III and Pst 1, respectively. One or three extra bases were added during primer synthesis on the 5′-ends of WA1 and WA2 or WA11 and WA12, respectively, to ensure proper reproduction of the restriction sites by Taq DNA polymerase during PCR and subsequent digestion. The primers WBS2 and WBS4 anneal specifically to pBS - DNA sequences flanking the multicloning site [Weier and Rosette 1988 and 1990].

b. Amplification of chromosome 10-specific alpha satellite DNA

Approximately 2,000 human chromosomes 10, isolated by flow sorting from the human X hamster cell line R342-A4 [Trask et al. 1991] were used as DNA template in the initial reaction. The reaction buffer consisted of 5 units of Taq DNA polymerase mixed with 200 ul amplification buffer [10 mM Tris-HCl, pH 8.4 at 20 °C, 1.5 mM $MgCl_2$, 50 mM KCl], 0.2 mM each dATP, dCTP, dGTP and dTTP, and 1.2 uM each of the primers WA1 and WA2, as described previously. Mineral oil [100 ul, Squibb, Princeton, NJ (USA)] was layered on top of the reaction mix to prevent evaporation during PCR. DNA amplification was performed during 45 cycles using a Perkin Elmer Cetus Thermal Cycler [Norwalk, CT (USA)]. Each cycle included a denaturation step of 120 sec at 94°C (180 sec. for the initial denaturation), primer annealing at 53°C for 60 sec. and primer extension for 120 sec at 72°C. Organic and aqueous phases were inverted in the reaction tube by addition of 1.5 volumes of chloroform, and PCR products were transferred to a clean tube. Amplification of alpha satellite DNA was confirmed visually by electrophoresis of a 10 ul aliquot of the PCR reaction in 4% agarose in 40 mM Tris-acetate, 1 mM EDTA, pH 8.0 containing 0.5 ug/ml EB [Maniatis et al. 1986]. The concentration of double stranded DNA in the reaction was determined by Hoechst 33258 fluorescence using a TK 100 fluorometer according to the manufacturer's protocol [Hoefer Scientific, San Francisco, CA (USA)].

Gel electrophoresis of unlabeled chromosome 10-specific PCR products from the initial amplification reaction using primers WA1 and WA2 revealed a strong band at approximately 175 bp indicating the successful amplification of the alphoid monomer sequences (Fig. 2, lane 1). Higher molecular weight DNA fragments were generated with much lower efficiency and appeared as a band at approximately 346 bp with an underlying smear of high molecular weight DNA when larger amounts of PCR products were loaded on the gel (data not shown). Separation of the products on 1.8% agarose showed that the background of heterogeneously sized DNA fragments in the range of 600 bp to 20 kb.

### c. Labeling of Chromosome 10-specific alphoid DNA

A four microliter aliquot of the PCR solution from the initial reaction was resuspended in 200 ul of biotinylation Buffer A wherein dTTP is replaced with Biotin-11-dUTP, and there is 1.2 uM of each primer WA1 and WA2 and 8 units of Taq. The mix was overlaid with 100 ul of mineral oil and alphoid DNA was amplified and biotinylated for an additional 20 cycles. Mineral oil was removed after addition of chloroform, and labeled probe DNA was stored without further purification at minus 20°C until used for in situ hybridization.

Aliquots of 200 ul from the initial PCR reactions were labeled with AAF with minor modifications of the procedure published by Landegent et al. (1984) as described in Weier et al. (1991b). After the reaction, DNA was extracted three times with phenol/chloroform/isoamyl alcohol [Maniatis et al. 1986] and then ether-extracted twice at room temperature. DNA was then precipitated in 2.5 volumes of ethanol, 0.1M Na acetate, dried and resuspended in 500 ul 10 mM Tris-HCl, pH 8.0, 1mM EDTA.

Labeling of PCR products with digoxigenin was performed in a manner similar to the described biotinylation reaction, except that the labeling buffer contained a mixture of digoxigenin and dTTP (0.16 mM and 0.04 mM, respectively) in place of the Biotin-11-dUTP.

### d. Synthesis of alphoid blocking DNA

Unlabeled total human alphoid DNA was synthesized from male human genomic DNA as described elsewhere [Weier et al. 1991a]. Briefly, 200 ng of isolated genomic DNA were mixed with 100 ul amplification mix containinig WA1 and WA2 as described above. PCR was performed for 30 cycles, and the concentration of double stranded DNA was determined by fluorometry. Human placental DNA was prepared for use as a blocking agent by sonication of the supplied DNA until the average size was approximately 300-400 bp as judged by agarose gel electrophoresis.

### e. Sample Preparation

Metaphase spreads were made from phytohemagglutinin-stimulated short-term lymphocyte cultures according to the procedure described by Harper et al. (1981b). Acetic acid/methanol (1:3, Carnoy's fixative) fixed metaphase spreads were prepared as described elsewhere [Weier et al. 1990]. Slides were stored under dry nitrogen in sealed plastic bags at -20°C until used. Cellular and chromosomal DNA was thermally denatured prior to application of the probe mixtures by incubating the slides for 4 min. at 74°C in 70% formamide, 2xSSC, pH 7.0. The slides were then dehydrated in a 70, 90, and 100% ethanol series and briefly air dried at room temperature.

Samples of kidney tissues were obtained from patients with renal cell carcinoma (RCC) after surgical removal of the tumor bearing kidney. Fresh samples originating from normal tissue adjacent to the primary tumors were washed twice in phosphate buffered saline (PBS) and were then placed in 5 ml of 30 mM EDTA. The tissues were minced with a scalpel and filtered through a 54 um nylon mesh [Kunicka et al. 1987]. The cell suspension was centrifuged at 600 g for 5 minutes, the supernatant was discarded, and the pellet was fixed in freshly prepared Carnoy's for 20 minutes at room temperature. Microscope slides were cleaned as described elsewhere [Weier et al. 1991a]. The fixed cells were further washed in two changes of Carnoy's and dropped on the slides. Touching imprints were prepared from frozen tissue samples by pressing the sample against clean glass slides without pretreatment. Cells adhering to the glass were fixed immediately in Carnoy's for 20 minutes.

Pretreatment of fixed kidney cell nuclei was performed with pepsin (100 ug/ml in 0.01 N HCl) for 10 minutes at room temperature [Hopman et al. 1989]. The interphase cells were further fixed by a 10 minute immersion in paraformaldehyde [4% (w/v)in PBS]. The slides were then washed in 2xSSC and denatured in 70% formamide, 2xSSC, pH 7.0 for 10 minutes at 72°C. Slides were then dehydrated in a 70%, 80%, and 100% ethanol series, air dried and prewarmed to 37 °C prior to addition of the probe mixture.

### f. In Situ Hybridization

Labeled probe DNAs (approximately 20 ng) and selected amounts of unlabeled human genomic DNA or total human alpha satellite DNA were added without purification to 8 ul of the hybridization mixture described by Pinkel et al. (1986). Water was added to 10 ul when necessary, so that the final concentration in the hybridization mixture was 55% formamide, 10% dextran sulfate, 1 ug/ul herring sperm DNA, 2xSSC, pH 7.0. DNA in the hybridization mix was denatured at 74°C for 5 minutes. After chilling on ice, the mix was added to the heat denatured slides, covered by a 22 mm by 22 mm coverslip and hybridized overnight at 37°C. The slides were then washed in 50% formamide, 2xSSC, pH 7.0 at 42°C for 15 minutes, followed by 2 washes of 15 minutes in PN buffer at 37°C. Biotinylated probe was detected with a 20 minute incubation at RT in avidin-FITC (5 ug/ml in PNM buffer). Excess avidin-FITC was removed by washes in two changes of PN buffer at RT, and the DNA was counterstained with PI or DAPI at a concentration of 1 ug/ml or 0.5 ug/ml, respectively, in 1% p-phenylenediamine, 15 mM NaCl, 1 mM $H_2PO_4$, pH 8.0, 90% glycerol [antifade solution, Johnson and de C. Noqueira Araujo 1981] for metaphase chromosome identification.

Paraffin embedded tissue sections of 4 um thickness were deparaffinized in preparation for in situ hybridization following the protocol of Brigati et al. (1983) that includes the sequential applications of pronase and HCl for tissue processing. The sections were then denatured for 20 min. in 70% formamide, 2xSSC at 74°C, dehydrated in a 70%, 80%, 100% ethanol series and hybridized as described above. Post hybridization washes of tissue sections were extended to three times 30 min. in 50% formamide, 2xSSC at 42°C, followed by three washes in PN buffer at 37°C. The tissue sections were then incubated for 20 min. with avidin-FITC as described above, washed in four changes of PN buffer of 15 min. each and mounted in antifade containing 0.5 ug/ml PI.

AAF-labeled probe DNA was detected after incubation with 10 ul of the supernatant from two murine cell lines (MBL4F and MBL6B) that produce monoclonal antibodies against AAF. [Those two cell lines were kindly provided by Dr. R. Baan, TNO, Rijswijk, The Netherlands.] The slides were then washed twice in PN buffer at RT, incubated for 15 min. at RT with a 1:25 dilution of the fluoresceinated goat anti-mouse antibodies in 1xPBS (Mg, Ca free), 2% normal goat serum, 0.05% Tween 20, and washed in two changes of PN buffer at RT.

The detection of digoxigenin-labeled DNA was done with the antibodies against digoxigenin. In these experiments, the digoxigenin-labeled probe was hybridized simultaneously with a biotinylated probe. In some experiments, the uncloned biotinylated chromosome 10 probe was hybridized with a digoxigenin-labeled DNA. The Alu DNA probe was generated from a cloned Alu repeat DNA fragment in a procedure similar to that described herein. The simultaneous hybridization of Alu probes is helpful for chromosome identification due to preferential binding of such probe to specific chromosomal regions in a manner very similar to R-bands [Baldini and Ward 1991]. In other experiments, digoxigenin-labeled probes generated from clonal chromosome 10-specific template DNA were hybridized with the Alu probe labeled with biotin or digoxigenin, respectively.

Slides were washed in the post-hybridization solutions as described above and blocked by incubation with PNM for 10 minutes at RT. Equal volumes of the fluoresceinated anti-digoxigenin antibody (20 ug/ml in PNM) and avidin-Texas Red (2 ug/ml in PNM) were mixed and applied to the slides. Incubation was performed under a coverslip for 25 minutes at RT in the dark. The slides were then washed in two changes of PN buffer at 37°C and mounted in DAPI in antifade solution.

Microscopy was performed on a Zeiss STANDARD fluorescence microscope (Zeiss, Oberkochen, FRG) equipped with a Plan-Neofluar 63x/1.20 Oil objective using the epi-illumination filter set for FITC that allows simultaneous observation of PI fluorescence or a filter set for simultaneous observation of FITC and TR [Omega Optical, Brattleboro, VT (USA)]. Photographs were recorded on Kodak Ektachrom 400 film.

### g. PCR Product cloning

PCR products were amplified for cloning into the Bam H1 site of pBS vector [Stratagene, San Diego, CA (USA]) by resuspending appoximately 12,000 flow sorted human chromosomes 10 (1530 per ul) in 400 ul reaction buffer containing primers WA11 and WA12 (shown in Table I) (1.2 uM) in the presence of the four unmodified dNTPs (0.25 mM each) and 8 units of Taq polymerase.

The thermal cycler was programmed to perform an initial 6 cycles with a primer annealing temperature of 40°C, followed by 29 amplification cycles with primer annealing at 50°C. Following DNA amplification, one tenth volume of 5 M Na acetate was then added, and the DNA was precipitated in an equal amount of isopropanol, washed with 70% ethanol, air dried and resupended in 20 ul of 1x Bam HI digestion buffer containing 20 units of Bam HI. The reaction was incubated at 37°C for 30 min., the DNA was precipitated in 2-propanol and the pellet was washed with 70% ethanol. The PCR products were then resuspended in 100 ul water. Two microliters of the DNA fragments were then resuspended in 30 ul 1x ligase buffer containing 10 units T4 DNA ligase and approximately 1 ug Bam HI-digested pBS DNA. The ligation was done at 15°C overnight. A 1 ul aliquot of the ligation reaction was diluted 1:5 with 1xTE buffer (10mM Tris-HCL, 1 mM EDTA, pH 8.0). One microliter of this dilution was used to transform competent DH5 alpha cells according to the supplier's protocol. Bacterial cells were then diluted 1:10 in Luria-Bertani (LB) medium [Maniatis et al. 1986] containing 100 ug/ml ampicillin, and plated on LB agar containing 100 ug/ml ampicillin, 4 ug/ml IPTG and 40 ug/ml X-Gal.

### h. Library Screening by PCR

Individual white colonies were picked by using sterile pipette tips and grown in LB broth containing 100 ug/ml ampicillin. A modification of the multiplex PCR protocol [Chamberlain et al. 1888] wherein one pair of primers was used to amplify a number of different DNA sequences was applied. PCR was performed by combining and resuspending four 1 ul aliquots from different overnight cultures in 50 ul amplification buffer containing the primers WBS2 and WBS4, the four unlabeled dNTPs, salts and Taq polymerase as described above for the initial amplification. PCR was performed for 40 cycles with primer annealing at 53°C. Clones that contained PCR products in the desired size range were then individually amplified in 40 cycle PCR reactions by resuspending 1 ul aliquots from the bacterial cultures in 40 ul reaction buffer containing either primer pair WBS2 and WBS4, or WA11 and WA12. Gel electrophoretic analysis of 8 ul aliquots was done in 4% agarose with 200 ng sizemarker DNA ($\phi$X174 RF DNA/Hae III digest) in separate lanes.

### i. Generation of Labeled Probes from Bacterial Clones

Amplification products (1 ul) of reactions using cells from representative clones, pBS609-51 and pBS609-52 with the vector-specific primers WBS2 and WBS4 were resuspended in biotinylation buffer containing the insert-specific primers WA11 and WA12. Labeling of the DNA fragments was performed in a 20 cycle PCR as described above. The labeling and simultaneous amplification of PCR products with digoxigenin was performed during 20 cycles similar to the biotinylation reaction, except that the digoxigenin-labeling buffer contained a mixture of digoxigenin-11-dUTP and dTTP (0.16 mM and 0.04 mM, respectively) instead of Biotin-11-dUTP.

In situ hybridization of biotinylated DNA was done as described for the uncloned probe. One microliter of digoxigenin-labeled DNA was mixed with 1 ul of a biotinylated Alu DNA probe, 8 ul MasterMix 2.1 (MasterMix 2.1 is 78.6% formamide, 14.3% dextran sulfate, 2.9 xSSC, pH 7.0) and 1 ul herring sperm DNA (10 mg/ml).

### j. Probe DNA Sequencing

Plasmid DNA was isolated from 500 ml overnight cultures of bacterial clones in LB-medium using the Maxiprep columns [Qiagen, Studio City, CA (USA)] according to the manufacturer's instructions. DNA sequencing was performed by dideoxynucleotide sequencing reactions according to the protocol described by Sanger et al. (1977). Double stranded DNA was sequenced by extension of T3- or T7-primers with dATP, dCTP, dTTP and either dGTP or dITP in the reaction buffer. DNA sequences were read from the original X-ray films. Further processing of the DNA sequences was done using the GeneWorks software obtained from IntelliGenetics, Inc. [Mountain View. CA (USA)].

### Results

### k. In Situ Hybridization of Degenerate Probe DNA in the Presence of blocking DNA

Hybridization of a biotinylated chromosome 10-specific probe to normal metaphase spreads in the absence of blocking DNA showed labeling of the centromeric regions on numerous chromosomes (Fig. 1A). Interphase cell nuclei typically showed several fluorescent domains (Fig. 1B).

The addition of 100 ng of total human alphoid blocking DNA to the hybridization mixture containing approximately 20 ng of biotinylated probe DNA reduced the crosshybridization so that the target chromosomes 10 could easily be identified by their brightly labeled centromeric region (Fig. 1C). Under these conditions, domains containing chromosome 10-specific centromeric DNA could easily be observed and counted in interphase cell nuclei (Fig. 1D). The effect was very similar when 500 ng of human genomic DNA were added to the hybridization cocktail.

The competitive hybridization regimen using human genomic DNA was used for determining the number of chromosomes 10 in single cell suspensions of kidney tissue adjacent to a surgically removed tumor. The hybridization mixtures typically contained 20 ng of AAF-labeled probe DNA and 500 ng of unlabeled human genomic DNA. Results of the hybridization of the AAF-labeled probe to cell samples from a renal cell carcinoma patient with a tumor karyotype of 47, XY, +10 are depicted in Figures 1D and 1E. Cells shown in Figure 1D showed two bright domains representing chromosome 10-specific alphoid DNA. The interphase cell in Figure 1E, however, showed three yellow fluorescent domains indicating the presence of an extra copy of chromosome 10 (arrows). Thus, in this particular preparation of single cells, the relative fraction of karyotypically abnormal cells could rapidly be assessed with an approximate error margin of a few percent by counting cells with three domains.

The degenerate probe DNA of this example was applied in a hybridization to deparaffinized tissue sections. The overnight incubation with biotinylated probe was done in the presence of unlabeled human genomic DNA to block crosshybridization. However, only low levels of crosshybridization was observed when the blocking DNA was omitted. As shown in Figure 1F, domains of bound probe DNA could be observed and counted after application of avidin-FITC.

1. Molecular cloning of the PCR Products and Sequence Analysis.

Recombinant clones were identified as white colonies on ampicillin plates, picked and grown overnight in LB medium containing ampicillin. 64 colonies were selected for further analysis. Bacterial clones were analyzed by multiplex PCR using the vector-specific oligonucleotides WBS2 and WBS4. Gel electrophoresis showed different sized amplification products for several samples. The size of the insert can be derived from the product size by substracting 114 bp, i.e., the distance of the 5'-ends of the primers when annealed to pBS DNA.

In situ hybridization was performed with probes that were generated by PCR amplification and labeling using the bacterial clones as DNA templates. The biotinylated probes (Fig. 1G and H) as well as the digoxigenin labeled DNA probe (Fig. 1I) showed high signal intensities and specificity with interphase nuclei and metaphase chromosomes. The intensity on the target chromosomes 10 was at least as high as observed using the degenerate probe;

there was, however, no sign of crosshybridizaton with other chromosomes.

DNA sequencing was performed on DNA isolated from clones pBS609-51 and pBS609-52. The results of DNA sequence analysis are shown in Table II. The sequencing reactions revealed inserts of 191 bp in the Bam H1 sites of pBS609-51 and pBS609-52, respectively. Both inserts are alpha satellite DNA repeat monomers flanked by the PCR primer sequences, and have 87% (pBS609-51) and 88.3% (pBS609-52) homology with the alphoid consensus monomer (Willard and Waye 1987). When 162 bp excluding the 5'-polylinkers were compared with a tetrameric alphoid DNA fragment reported to be chromosomes 12-specific (pBR12; Baldini et al. 1990; EMBL/GenBank accession number M28221), the clones pBS609-51 and pBS609-52 showed high homology with individual monomers. As much as 83.3% (pBS609-51) and 84.6% (pBS609-52) sequence homology was found with the second monomer of pB12 (Table II). The GenBank/EMBO accession numbers for those sequences are X67271 (pBS609-51) and X67272 (pBS609-52).

The results of cloning experiments of chromosome 10-derived alphoid DNA presented in this example suggest the presence of one or several specific monomer sequences. The simple cloning strategy employed for isolation of chromosome 10-specific DNA produced a number of recombinants containing alpha satellite DNA inserts. However, analyses of 64 randomly selected recombinants indicated that there were many small inserts that provide primer annealing sites and possibly represent the small fragments amplified with primers WA11 and WA12 (Fig. 2, lane 3). Among the 64 clones, four clones were found that contained alphoid monomers and that hybridized relatively specifically with human chromosome 10.

The library screening procedure using PCR and bacterial cells [Weier and Rosette 1990] is very rapid and efficient. The high sensitivity of PCR allows the pooling and amplification of DNA from more than 4 clones in one reaction (Green and Olsen 1990; Weier et al. 1991c). For rapid screening of colonies, an accelerated protocol similar to the procedure described by Gussow and Clackson (1989) can be used. This scheme allows generation of novel hybridization probes in less than two weeks starting with appropriate oligonucleotides and amplification templates.

Table II: DNA sequences of the alphoid consensus mono-mer[1], the two PCR-isolated chromosome 10-specific alpha satellite DNA clones and four alpha satellite monomers specific for the human chromosome 12[2].

| | | | | |
|---|---|---|---|---|
| Primer WA1 | | GAAGCTTA$^A_T$ $^G_CT^A_C$ACAGAGT | T$^G_T$AA | |
| CONSENSUS | | AA | CTCACAGAGT | TGAAC$^A_C$TT$^T_C$C | TTTT$^G_C$ATAGA |
| pBS609-51[3] | GGATCCCTGC AGAAGCTTAA | GTCACAGAGT | TGAACCTTCC | TTTAGACAGA |
| pBS609-52[3] | GGATCCCTGC AGAAGCTTAT | GTAACAGAGT | TGAACCTTCC | TTTAGACAGA |
| I | | CAA | CTCAAGGTGT | TTAAGCTTTC | TTTTCATAGA |
| II | | CAA | CTCACAGAGG | TGAACTGTCC | TTTAGACAGA |
| III | | CAA | TTCACAGAGA | TAACCTTTCT | TTT-GATGAA |
| IV | | CAA | CTCACAGAGT | TGAACCTTCC | TTTAGACAGA |

| | | | | |
|---|---|---|---|---|
| CONSENSUS | GCAGTTT$^G_T$GA | AACAGTCTTT | TTGTAGAATC | TGCAAGTGGA | $^T_CATTTGGA^G_C$C |
| pBS609-51 | GCAGTTTTGA | AAAATTCTTT | CTGTGGAATC | TGCAAGTGGA | GATTTCAAGC |
| pBS609-52 | GCAGTTTTGA | AAAACTCTTT | CTGTGGAATT | TGCAAGTGGA | GATTTCAAGC |
| I | GTAGTTTGGA | AACACTCTGT | CTGTAAAGTC | TGCAAGCAGA | TATTTGGACC |
| II | GCAGATGTGA | AACCCTCTTT | TTGTGATATT | TGCAGGTGGA | GATTTCAAGC |
| III | GGAGTTTGGA | GACACTGTGT | TTGTAAAGTC | TGCAAGTGGA | TATTTGGACC |
| IV | GCAGATTTGA | AACACCCTAT | TTGTGCAGTT | TCCAGTTGGA | GATTTCAATC |

| | | | | |
|---|---|---|---|---|
| CONSENSUS | $^G_TCTTTGAGG^A_C$ | $^T_CT^A_T^T_CG^G_TTGGA$ | AA$^A_CGG^G_AAATA$ | TCTTCA$^T_CATA$ | AAA$^A_-CTA^G_AAC$ |
| pBS609-51 | GATTTGAGGC | TAATCTTTGA | AATGGAAATA | TCCTCGTGTA | AAAACTACAC |
| pBS609-52 | GATTTGAGGC | TAATCTTTGA | AATGGAAATA | TCTTCGTGTA | AAAACTACAC |
| I | TCTTTGGGGC | CTTCGTTGGA | AACGGG-ATT | TCTTCATAGA | A-CGCTAGAA |
| II | GCTTTTAGGC | CAAATGTAGA | AAAGGAAATA | TCTTCGTATA | AAAACTAGAC |
| III | TCTTTGAGGC | CTTCGTTGGAAGGAAACGGGATT | TCTTCCTGTA | A-TGTTCGAC |
| IV | ACTTTGAGAC | CAAATGTACA | AAAGGAAACA | TCTTCGTATA | AAAACTAGAC |

| | | | |
|---|---|---|---|
| Primer WA2 | | GAAACT$^T_AG$ CTT$^G_TG^G_TGATC$ | TGCAGC |
| CONSENSUS | AGAAGCATTC | TCAGAAACTT | CTTTGTGAT |
| pBS609-51 | AGAATCATTC | TCAGAAACTA | CTTGGGGAT**C TGCAGAAGCT TGGATCC** |
| pBS609-52 | AGAATCATTC | TCAGAAACTT | CTTGGTGAT**C TGCAGAAGCT TGGATCC** |
| I | AGAAGAATAC | TGAGTAAGTT | CTTTGTGTTGCCTCTATT |
| II | AGAATCATTC | TCAGAAACTA | CTTTGTGATGTGTGCGTT |
| III | AGAAGAATTC | TCAGTAACTT | ATTTGTGGTGTGTGTATT |
| IV | AGAATCATTC | TCAGAAACTA | CTTTGTGATGTGTGCGTT |

[1] The human alpha satellite monomer consensus sequence as defined by Willard and Waye (1987).
[2] I-IV: The chromosome 12-specific alphoid monomer sequences published by Baldini et al. (1990).
[3] The underlined bases represent nonhomologous 5'-polylinker regions of WA11 and WA12, resp.

Example 2

Probes that are specific for human chromosome 8-specific alpha satellite DNA were prepared very similarly to the procedures detailed in Example 1. The inventors' work with those chromosome 8-specific probes is reported in Weier et al., Hum. Genet., 87: 489-494 (1991).

Example 3

Chromosome-specific Centromeric Probes for Chromosome 17 and Chromosome 3

The procedures essentially as described in Example 1 were used to prepare two centromere-specific alpha satellite probes; one for the centromeres of chromosome 17, and one for the centromeres of chromosomes 3. The WA1 and WA2 primers were used, and approximately 50 ng of DNA from chromosome 17 was used as the DNA template. The DNA template was isolated from the Bluescribe plasmid library for chromosome 17 (pBS17), which in turn had been prepared by subcloning an entire chromosome 17 library that is publicly available as deposit libraries LL17NS01 or LA17NS03 [Van Dilla et al. 1986].

The DNA amplification and simultaneous biotinylation was performed during 45 cycles using an automated thermal cycling system [Weier and Gray 1988] with a thermal denaturation step of 90 seconds at 94°C (120 seconds for the initial denaturation). Primer annealing during the second step of each cycle was performed at 53°C for 90 seconds. The temperature was then increased slowly (7°C/minute) to 72°C. The cycle was completed by holding that temperature for 120 seconds for primer extension.

A probe specific for alpha satellite centromeric repeats on human chromosome 3 was similarly prepared by in vitro DNA amplification using WA1 and WA2 primers and approximately 80 ng of CsCl gradient isolated DNA from the Bluescribe plasmid library for chromosome 3 (pBS3) (400 ng/ml) as amplification DNA template. PCR was performed for 30 cycles using an automated thermal cycler [Perkin-Elmer/Cetus, Norwalk, CT (USA)]. The DNA template was denatured at 94°C for 1 minute. Primer annealing and extension were performed at 53°C and 72°C, respectively. Probe biotinylation occurred in the presence of Biotin-11-dUTP and further amplification was accomplished during an additional 20 PCR cycles.

## Example 4

### Centromere-Specific Probes for Chromosomes 21 and 13

A 13/21 centromeric probe was prepared by PCR according to procedures essentially as described in Example 1 and Weier et al. (1991a) except that nondegenerate primers were used. Briefly, the probe was made by PCR using flow sorted human chromosome 21s as a template and two primers (30 uM) specific for the alphoid sequence--primers W21R1 and W21R2 as shown in Table I, supra. The product was labeled during the PCR reaction by including biotin 11-dUTP (100%). The oligonucleotide primers were synthesized and purified as shown in Example 1. Using the flow sorted chromosome DNA as a template, a 135 bp product was generated.

## Example 5

### Chromosome-Specific Probe for a Repeat Sequence on Chromosome 9

Probes which bind exclusively to chromosome 9 were prepared by selecting oligonucleotide primers WYR9 and WYR10 (shown in Table I) which bind to a pentameric repeat motif, TTCCA, of the satellite III DNA which is found as major blocks on human chromosomes 1, 7, 9, 15, 16, 17, 21, 22 and Y [Berdize 1985]. WYR9 and WYR10 bind respectively at positions 2813-2840 and 2777-2743 in Y chromosome specific 3.4 kb satellite III repeat sequence [Nakahori et al. 1986].

The primers were amplified by PCR with either male or female human genomic DNA or isolated chromosome 9 DNA as the template DNA. Probe DNA amplified with the male human genomic template hybridized strongly with repeated DNA on the long arm of the Y chromosome, and on the satellite III region of chromosome 9 as well as several other chromosomes. When female human genomic DNA was the template, the produced probes hybridized with similar autosomal DNAs but only showed weak crosshybridization with the Y chromosome.

When isolated chromosome 9 was the template, the probe produced bound to chromosome 9 at the 9q12 position with negligible crosshybridization with other chromosomes. At least in this example, the primers used during PCR are less significant for binding specificity that the nature of the DNA template used.

## Example 6

### Chromosome-Specific Probe for a Repeat Sequence on Chromosome X

Oligonucleotide primers, WXR1 and WXR2 as shown in Table I, were selected which flank a 70 bp segment from position 1595 to position 1664 in the 2.0 kb pair of the X chromosome-specific alpha satellite DNA repeat sequence [Waye et al. 1985]. The primers were synthesized and purified as indicated in Example 1. The probe produced by PCR amplification of primers WXR1 and WXR2 is 124 bp.

The probe produced binds exclusively to the X chromosome and can be used for rapid identification of that chromosome, especially in fetal tissues. That probe may also be used to monitor the progress of bone marrow transplantation of sex-mismatched donor and recipient or to determine the presence of X chromosomes in human sperm.

Example 7

Mouse Centromeric Probes

The oligonucleotide primers WGS1 and WGS2 (Table I), which anneal in the conserved region of the 234 bp gamma satellite repeat of mouse DNA were selected to amplify gamma satellite repeat probes. Primer annealing sites were chosen to be in close proximity within the gamma satellite DNA consensus sequence (Vissel and Choo 1989). More than half of the published 30 cloned gamma satellite monomers allow the primers to anneal with their respective 3′ ends in a distance of 14 bp. PCR was expected to amplify preferentially 55 bp DNA fragments.

Gel electrophoretic analysis of PCR products wherein mouse total genomic DNA was the template showed a strong band of unlabeled PCR products at about 290 bp. Further work of the inventors with gamma satellite DNA can be found in Weier et al. (1991c).

The foregoing description of preferred embodiments of the invention has been presented for purposes of illustration and description. It is not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated.

All references herein cited are hereby incorporated by reference.

## Claims

1. A method of preparing chromosome-specific repeat sequence nucleic acid probes comprising:

   binding a first set of degenerate oligonucleotide primers to repeat sequence units in a template DNA that is chromosome-specific;

   binding a second set of degenerate oligonucleotide primers to said repeat sequence units such that each of the 5′ ends of said first set of primers faces a 5′-end of one of said second set of primers and the binding sites of said first set of primers are within a distance of between about 20 bp to about 5 kilobases (kb) of the binding sites of said second set of primers; and

   amplifying the template DNA by a polymerase chain reaction (PCR) method between and including the first and second primers to produce chromosome-specific repeat sequence nucleic acid probes.

2. A method according to Claim 1 wherein the repeat sequence units are clustered and are

   alpha satellites, beta satellites, satellites I, II, III or IV or the 39 bp repeat that maps to 1p36.

3. A method according to Claim 1 wherein said first set of degenerate oligonucleotide primers is the same as said second set of oligonucleotide primers.

4. A method according to Claim 1 wherein the primer set is Jun1.

5. A method according to Claim 2 wherein the repeat sequence units are alpha satellite monomers and wherein the first and second set of degenerate primers are WA1 and WA2 and/or WA11 and WA12.

6. A method according to Claim 1 wherein the chromosome-specific template DNA has been isolated by flow-sorting, by microdissection, or by density gradients; is in a hybrid cell; or is from a chromosome-specific library.

7. A method according to Claim 6 wherein the chromosome-specific DNA is flow-sorted chromosomes selected from human chromosomes 1 through 22, X and Y.

8. A method according to Claim 1 wherein said method further comprises the step of incorporating modified dNTPs during the amplifying step.

9. A method according to Claim 1 further comprising the step of labeling the repeal sequence probes after the amplifying step is completed by chemically or enzymatically modifying the probe molecules.

10. A method according to Claim 1 further comprising the step of inserting said probes into a cloning vector after said amplification step and cloning said probes.

11. A method according to Claim 10 wherein the produced clones are screened to find clones that are highly specific to a single chromosome.

12. A method according to Claim 11 wherein clones that are found to be highly specific for said repeated sequences are used as clonal templates for PCR amplification.

13. A chromosome-specific repeat sequence probe prepared by the method of any preceding claim and labeled during the amplifying step or thereafter.

14. A method of preparing chromosome-specific repeat sequence DNA probes by arbitrary selection of repeat DNA sequences from human genomic DNA comprising the steps of:
    annealing to human genomic template DNA containing CAGG repeat sequences, the degenerate primer Jun1; and
    amplifying said template DNA by a polymerase chain reaction (PCR).

15. A method according to Claim 14 wherein said template DNA is chromosome-specific.

16. A composition of matter comprising a degenerate alpha repeat sequence probe specific for chromosome-10 centromeres having the nucleotide sequence shown for the insert of pBS609-51 or pBS609-52 as shown in Table II.

17. A method to enumerate specific chromosomes in metaphase spreads, germ line or somatic cell interphase nuclei and/or micronuclei comprising the steps of:
    in situ hybridizing thereto, the chromosome-specific repeat sequence probe of Claim 13 wherein the repeat sequence unit is alpha satellite DNA;
    rendering visible said labeled probe; and
    counting the number of centromeric and/or pericentromeric regions that are stained by said labeled probe.

18. A method according to Claim 17 wherein during said hybridizing step, blocking DNA is used.

19. A composition of matter comprising a degenerate oligonucleotide set of primers selected from WA1, WA2, WA11, WA12, and Jun1.

20. A composition comprising a chromosome-specific repeat sequence probe that is
    a human centromere-specific nucleic acid probe for human chromosomes 21 and 13 prepared from chromosome 21-specific template DNA by a polymerase chain reaction (PCR) method wherein the primers are W21R1 and W21R2; a human chromosome X-specific repeat sequence probe prepared from human chromosome X-specific template DNA by a PCR method wherein the primers are WXR1 and WXR2; a human chromosome 9-specific repeat sequence probe prepared from human chromosome 9-specific template DNA by a PCR method wherein the primers are WYR9 and WYR10; or a mouse centromere-specific repeat sequence probe prepared from mouse total genomic template DNA by a PCR method wherein the primers are WGS1 and WGS2.

21. A composition comprising an oligonucleotide primer selected from W21R1, W21R2, WGS1, WGS2, WXR1, WXR2, WYR9 and WYR10.

FIG. 1

FIG. 2

FIG. 3

EP 0 511 750 A1

European Patent Office

# EUROPEAN SEARCH REPORT

Application Number

EP 92 30 3159

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | CHROMOSOMA<br>vol. 100, no. 6, July 1991, BERLIN<br>pages 371 - 376;<br>WEIER, H-U. ET AL: 'TWO-COLOR HYBRIDIZATION WITH COMPLEXITY CHROMOSOME-SPECIFIC PROBES'<br>* the whole document *<br>--- | 1-2,<br>5-13,<br>17-19 | C12Q1/68<br>C12P19/34<br>C07H21/04 |
| P,X | HUMAN GENETICS<br>vol. 87, no. 4, August 1991, BERLIN<br>pages 489 - 494;<br>WEIER, H-U. ET AL: 'LABELING OF THE CENTROMERIC REGION ON HUMAN CHROMOSOME 8 BY IN SITU HYBRIDIZATION'<br>* the whole document *<br>--- | 1-2,<br>5-13,<br>17-19 | |
| D,A | CHROMOSOMA<br>vol. 98, no. 4, August 1989, BERLIN<br>pages 259 - 265;<br>KOCH, J.E. ET AL: 'OLIGONUCLEOTIOE-PRIMING METHODS FOR CHROMOSOME-SPECIFIC LABELLING OF ALPHA SATELLITE ONA IN SITU'<br>* the whole document *<br>--- | 1-2,<br>5-13,<br>17-19 | |
| A | PROCEEDINS OF THE NATIONAL ACADEMY OF SCIENCE OF USA<br>vol. 87, September 1990, WASHINTON<br>pages 6634 - 6638;<br>LICHTER, P. ET AL: 'FLUORESCENCE IN SITU HYBRIDIZATION WITH Alu AND L1 POLYMERASE CHAIN REACTION PROBES'<br>* the whole document *<br>--- | 1-3 | TECHNICAL FIELDS SEARCHED (Int. Cl.5)<br><br>C12Q |
| A | WO-A-9 002 821 (CETUS CORPORATION)<br>* the whole document *<br>*especially page 3 ,lines 17-26*<br>--- | 1-2 | |
| | -/-- | | |

The present search report has been drawn up for all claims

| Place of search | Date of completion of the search | Examiner |
|---|---|---|
| THE HAGUE | 15 JULY 1992 | OSBORNE H.H. |

EPO FORM 1503 03.82 (P0401)

**European Patent Office**

# EUROPEAN SEARCH REPORT

Application Number

EP    92 30 3159
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CYTOMETRY<br>vol. SUP 4, 1990, NEW YORK<br>page 57;<br>WEIER, H-U ET AL.: 'GENERATION OF CHROMOSOME-SPECIFIC DNA PROBES BY IN VITRO AMPLIFICATION OF SORTED CHROMOSOMES.'<br>* abstract *<br>--- | 1-2,6-13 | |
| A | WORLD PATENTS INDEX LATEST<br>Week 9119,<br>Derwent Publications Ltd., London, GB;<br>AN 91-140560<br>& US-A-7 454 171 (NAT INST OF HEALTH) 10 May 1990<br>* abstract *<br><br>----- | 1-2 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 15 JULY 1992 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)